# EUROPEAN PATENT APPLICATION

(11) **EP 4 744 606 A1**
(43) Date of publication of application: **20.05.2026**
(21) Application number: 25216713.5
(22) Date of filing: 18.11.2025
(51) Int. Cl.: A61B 18/00, A61B 18/12, A61B 34/00

(54) **SETTING EVACUATION MOTOR SPEEDS FOR SURGICAL TOOL EVACUATION MODULES**

(30) Priority: 18.11.2024 US 202418951342
(71) Applicant: Cilag GmbH International, 6300 Zug (CH)
(72) Inventor: SWENSON, Rachael A., Cincinnati, 45242 (US); MINNELLI, Patrick J., Cincinnati, 45242 (US); ASHER, Ryan M., Cincinnati, 45242 (US); VOEGELE, Aaron C., Cincinnati, 45242 (US)
(74) Representative: Carpmaels & Ransford LLP

(57) **Abstract**

A surgical system is disclosed including an evacuation module and a controller. The evacuation module comprises a motor and a pump drivable by the motor to draw smoke from a patient. The controller is operable to receive a first input indicative of a type of a surgical instrument, receive a second input indicative of a power level of the surgical instrument, and set a motor speed of the motor based on the first and second inputs.

## Description

### BACKGROUND

The present disclosure relates to surgical systems and, more particularly, insufflation and smoke evacuation systems used in surgical systems.

During a surgical procedure involving an energy device, insufflators are often used to provide insufflation to a patient's body cavity and thereby enhance visibility and access to the same. During the surgical procedure, smoke can be generated at a surgical site as the energy device is utilized. Surgical smoke evacuators are configured to evacuate smoke, as well as fluid and/or particulate, from the surgical site.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 is a block diagram of a computer-implemented interactive surgical system, in accordance with at least one aspect of the present disclosure.
FIG. 2 is a diagram of various modules, including an energy module, an evacuation module, and an insufflation module, and other components that are combinable to customize modular surgical systems, in accordance with at least one aspect of the present disclosure.
FIG. 3 is the energy module of FIG. 2 and various surgical instruments usable therewith, in accordance with at least one aspect of the present disclosure.
FIG. 4A is a first illustrative modular surgical system configuration including a header module and a display screen that renders a graphical user interface (GUI) for relaying information regarding modules connected to the header module, in accordance with at least one aspect of the present disclosure.
FIG. 4B is an isometric view of the modular surgical system shown in FIG. 4A mounted to a cart, in accordance with at least one aspect of the present disclosure.
FIG. 5 is a second illustrative modular surgical system configuration including a header module, a display screen, two energy modules, and an evacuation module connected together and mounted to a cart, in accordance with at least one aspect of the present disclosure.
FIG. 6 is a block diagram of an example modular surgical system, in accordance with at least one aspect of the present disclosure.
FIG. 7 is a schematic depicting internal components of the insufflation module of FIG. 2, in accordance with at least one aspect of the present disclosure.
FIG. 8 is a schematic depicting internal components within the evacuation module of FIG. 2, in accordance with at least one aspect of the present disclosure.
FIG. 9 is a method of controlling the modular surgical system of FIG. 6, in accordance with at least one aspect of the present disclosure.
FIG. 10 is a schematic view of the internal components of an evacuation module, in accordance with at least one aspect of the present disclosure.
FIG. 11 is a schematic view of the internal components of an evacuation module, in accordance with at least one aspect of the present disclosure.
FIG. 12 illustrates a detailed view of an inlet port of the evacuation module of FIG. 8, in accordance with at least one aspect of the present disclosure.
FIG. 13 is a method of controlling the modular surgical system of FIG. 6, in accordance with at least one aspect of the present disclosure.
FIG. 14 is a block diagram of another example modular surgical system, in accordance with at least one aspect of the present disclosure.
FIG. 15 is a method of controlling the modular surgical system of FIG. 14, in accordance with at least one aspect of the present disclosure.

### DETAILED DESCRIPTION

Applicant of the present application owns the following U.S. Patent Applications filed concurrently herewith, the disclosure of each of which is hereby incorporated by reference in their entirety herein:

U.S. Patent Application No. 18/950,801, titled IMPROVED FILTER LIFE IN SURGICAL smoke EVACUATION SYSTEMS;

U.S. Patent Application No. 18/950,899, titled AIR MANAGEMENT IN SURGICAL SMOKE EVACUATION SYSTEMS; and

U.S. Patent Application No. 18/951,268, titled INTELLIGENT INSUFFLATION AND SMOKE EVACUATION.

The present disclosure relates to energy devices, insufflation systems for providing insufflation gas to a patient, and evacuation systems for evacuating smoke and/or other fluids and/or particulates from a surgical site.

Smoke is often generated during surgical procedures that use energy devices, which use energy to affect (treat) tissue. In an energy device, the energy is supplied by a generator. Energy devices include devices with tissue-contacting electrodes, such as an electrosurgical device having one or more radio frequency (RF) electrodes, and devices with vibrating surfaces, such as an ultrasonic device having an ultrasonic blade. For an electrosurgical device, the generator is configured to generate oscillating electric currents to energize the electrodes. For an ultrasonic device, the generator is configured to generate ultrasonic vibrations to energize the ultrasonic blade. Generators are further described herein, and an evacuation module is utilized to control an amount of smoke generated by the energy devices during use thereof.

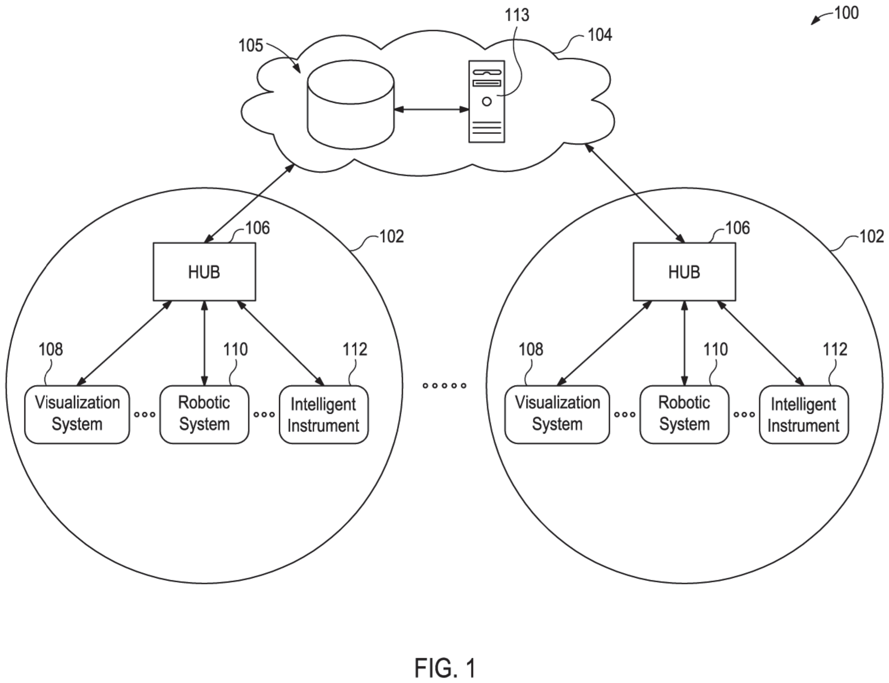

FIG. 1 is a block diagram of a computer-implemented interactive surgical system 100 (hereinafter "the surgical system 100") that may be used in accordance with at least one aspect of the present disclosure. The surgical system 100 includes one or more sub-surgical systems 102 and a cloud-based system (e.g., the cloud 104) that may include a remote server 113 in communication with a storage device 105. Each sub-surgical system 102 includes at least one surgical hub 106 in communication with the cloud 104 that may include a remote server 113.

In one example, as illustrated in FIG. 1, each sub-surgical system 102 includes a visualization system 108, a robotic system 110, and a handheld intelligent surgical instrument 112, which are configured to communicate with one another and/or the hub 106. In some aspects, each sub-surgical system 102 may include an M number of hubs 106, an N number of visualization systems 108, an O number of robotic systems 110, and a P number of handheld intelligent surgical instruments 112, where M, N, O, and P are integers greater than or equal to one. The surgical system 100 is described in more detail in U.S. Patent No. 11,666,368, entitled "METHOD FOR CONSTRUCTING AND USING A MODULAR SURGICAL ENERGY SYSTEM WITH MULTIPLE DEVICES", issued June 6, 2023, and is hereby incorporated by reference in its entirety herein.

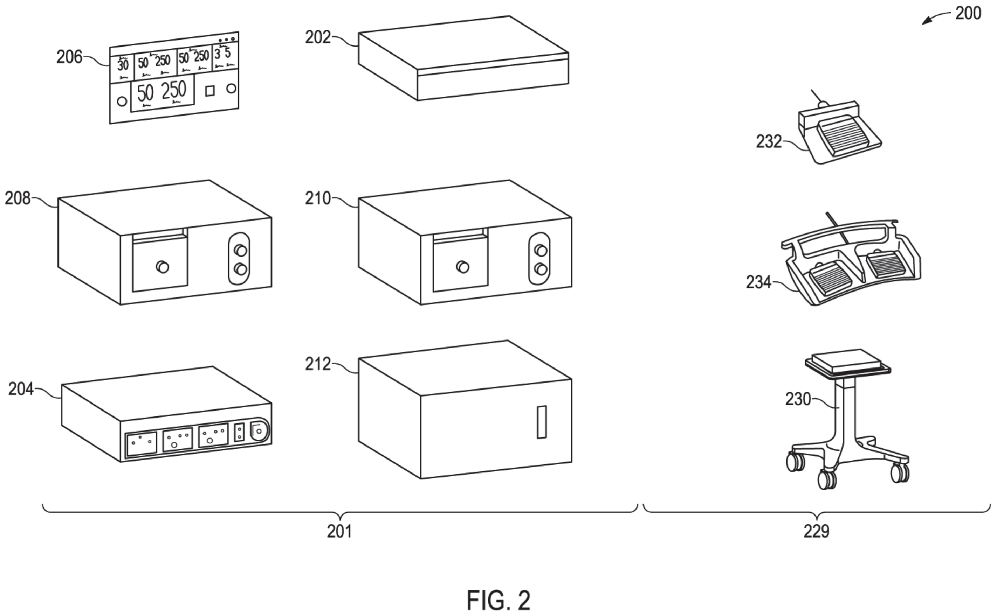

Referring now to FIG. 2, an example surgical hub 106 (FIG. 1) can be embodied as a modular surgical system 200 that can include a variety of different modules 201 that are connectable together in a stacked configuration. In one aspect, the modules 201 can be both physically and communicably coupled together when stacked or otherwise connected together into a singular assembly. Further, the modules 201 can be interchangeably connectable together in different combinations or arrangements. In one aspect, each of the modules 201 can include a consistent or universal array of connectors disposed along their upper and lower surfaces, thereby allowing any module 201 to be connected to another module 201 in any arrangement (except that, in some aspects, a particular module type, such as the header module 202, can be configured to serve as the uppermost module within the stack, for example). In an alternative aspect, the modular surgical system 200 can include a housing that is configured to receive and retain the modules 201. The modular surgical system 200 can also include a variety of different components or accessories that are also connectable to or otherwise associatable with the modules 201.

The modular surgical system 200 can be assembled from a variety of different modules 201, some examples of which are illustrated in FIG. 2. Each of the different types of modules 201 can provide different functionality, thereby allowing the modular surgical system 200 to be assembled into different configurations to customize the functions and capabilities of the modular surgical system 200 (e.g., by customizing the modules 201 that are included in each modular surgical system 200). The modules 201 of the modular surgical system 200 can include, for example, a header module 202 (which can include a display screen 206), an energy module 204, an evacuation module 208, an insufflation module 210, and a visualization module 212.

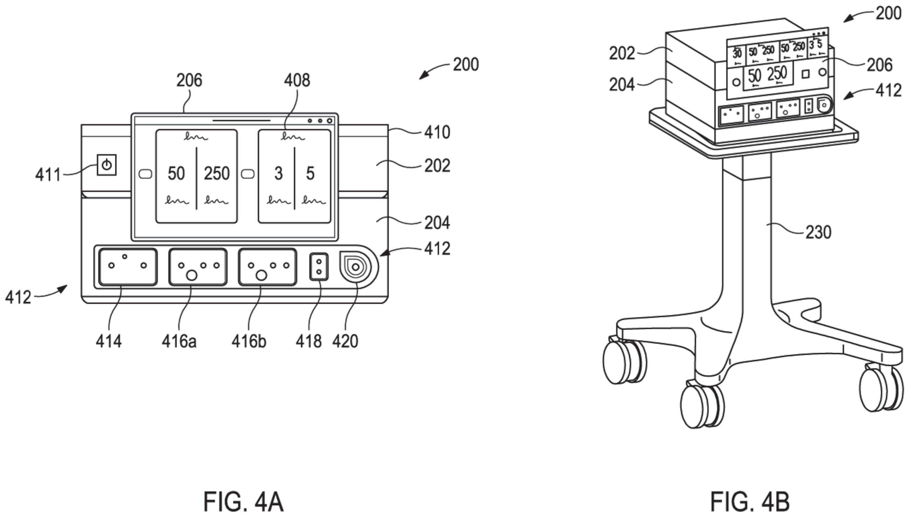

In the depicted aspect, the header module 202 is configured to serve as the top or uppermost module within the modular surgical system stack and can thus lack connectors along its top surface. In another aspect, the header module 202 can be configured to be positioned at the bottom or the lowermost module within the modular surgical system stack (*i.e.,* a "footer" module) and can thus lack connectors along its bottom surface. In yet another aspect, the header module 202 can be configured to be positioned at an intermediate position within the modular surgical system stack and can thus include connectors along both its bottom and top surfaces. The header module 202 can be configured to control the system-wide settings of each module 201 and component connected thereto through physical controls 411 (FIG. 4A) thereon and/or a graphical user interface (GUI) 408 (FIG. 4A) rendered on the display screen 206. Such settings could include the activation of the modular surgical system 200, the volume of alerts, the footswitch settings, the settings icons, the appearance or configuration of the user interface, the surgeon profile logged into the modular surgical system 200, and/or the type of surgical procedure being performed. The header module 202 can also be configured to provide communications, processing, and/or power for the modules 201 that are connected to the header module 202.

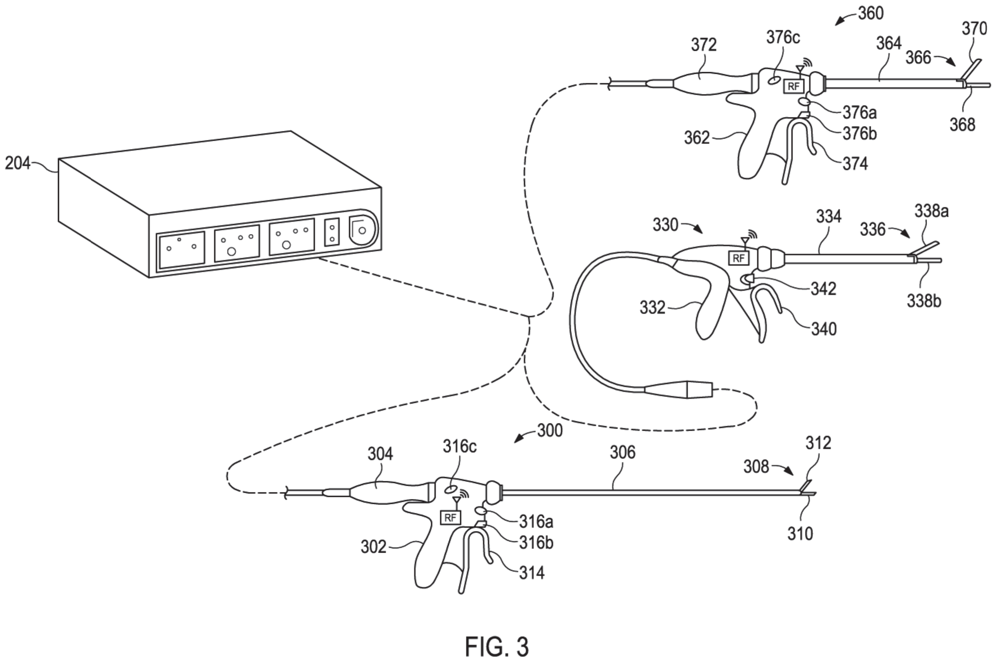

The energy module 204, alternately referred to as a generator module, can be configured to generate one or multiple energy modalities for driving electrosurgical and/or ultrasonic surgical instruments connected thereto. For example, referring to FIG. 3, the generator 204 is configured to drive multiple surgical instruments 300, 330, 360. The first surgical instrument is an ultrasonic surgical instrument 300 and comprises a handpiece 302 (HP), an ultrasonic transducer 304, a shaft 306, and an end effector 308. The end effector 308 comprises an ultrasonic blade 310 acoustically coupled to the ultrasonic transducer 304 and a clamp arm 312. The handpiece 302 comprises a trigger 314 to operate the clamp arm 312 and a combination of toggle buttons 316a, 316b, 316c to energize and drive the ultrasonic blade 310 or other function. The toggle buttons 316a-c can be configured to energize the ultrasonic transducer 304 with the generator 204.

The generator 204 is also configured to drive the second surgical instrument 330, which is an RF electrosurgical instrument and comprises a handpiece 332 (HP), a shaft 334, and an end effector 336. The end effector 336 comprises electrodes in clamp arms 338a, 338b and return through an electrical conductor portion of the shaft 334. The electrodes are coupled to and energized by a bipolar energy source within the generator 204. The handpiece 332 includes a trigger 340 manually actuatable to operate the clamp arms 338a,b, and an energy button 342 to actuate an energy switch to energize the electrodes in the end effector 336.

The generator 204 is also configured to drive the third surgical instrument 360, which is a multifunction surgical instrument 360 and comprises a handpiece 362 (HP), a shaft 364, and an end effector 366. The end effector 366 comprises an ultrasonic blade 368 and a clamp arm 370. The ultrasonic blade 368 is acoustically coupled to an ultrasonic transducer 372. The handpiece 362 includes a trigger 374 to operate the clamp arm 370, and a combination of toggle buttons 376a, 376b, 376c to energize and drive the ultrasonic blade 368 or other function. The toggle buttons 376a-c can be configured to energize the ultrasonic transducer 372 with the generator 204 and energize the ultrasonic blade 368 with a bipolar energy source also contained within the generator 204. Further aspects of the surgical instruments are described in U.S. Patent No. 10,624,691, entitled "TECHNIQUES FOR OPERATING GENERATOR FOR DIGITALLY GENERATING ELECTRICAL SIGNAL WAVEFORMS AND SURGICAL INSTRUMENTS", issued April 21, 2020, which is herein incorporated by reference in its entirety herein.

The evacuation module 208 (FIG. 2) can be configured to evacuate smoke, fluid, and/or particulates generated by the application of therapeutic energy to the tissue by one or more of the surgical instruments 300, 330, 360. Example evacuation modules are described in more detail elsewhere herein, as well as in U.S. Patent No. 11,602,393, entitled "SURGICAL EVACUATION SENSING AND GENERATOR CONTROL", issued March 14, 2023, which is hereby incorporated by reference in its entirety herein. The insufflation module 210 (FIG. 2) can be configured to blow air or gas into a body cavity of a patient to inflate it for diagnostic or surgical procedures, thereby providing better visibility and access during procedures.

The visualization module 212 (FIG. 2) can be configured to interface with visualization devices (i.e., scopes) and accordingly provide increased visualization capabilities. Example visualization modules and systems are described in more detail in U.S. Patent No. 11,284,963, entitled "METHOD OF USING IMAGING DEVICES IN SURGERY", issued March 29, 2022, which is hereby incorporated by reference in its entirety herein.

Referring again to FIG. 2, the modular surgical system 200 can further include a variety of accessories 229 that are connectable to the modules 201 for controlling the functions thereof or that are otherwise configured to work in conjunction with the modular surgical system 200. The accessories 229 can include, for example, a single-pedal footswitch 232, a dual-pedal footswitch 234, and a cart 230 for supporting the modular surgical system 200 thereon. The footswitches 232, 234 can be configured to control the activation or function of particular energy modalities output by the energy module 204, for example.

By utilizing modular components, the depicted modular surgical system 200 provides a surgical platform that grows with the availability of technology and is customizable to the needs of the facility and/or surgeons. Further, the modular surgical system 200 supports combo devices (e.g., dual electrosurgical and ultrasonic energy generators) and supports software-driven algorithms for customized tissue effects. Still further, the surgical system architecture reduces the capital footprint by combining multiple technologies critical for surgery into a single system.

The various modular components utilizable in connection with the modular surgical system 200 can include monopolar energy generators, bipolar energy generators, dual electrosurgical/ultrasonic energy generators, display screens, and various other modules and/or other components described elsewhere herein.

Referring now to FIG. 4A, the header module 202 can, in some aspects, include the display screen 206 that renders a GUI 408 for relaying information regarding the modules 201 (FIG. 2) connected to the header module 202. In some aspects, the GUI 408 of the display screen 206 can provide a consolidated point of control of all of the modules 201 making up the particular configuration of the modular surgical system 200. In alternative aspects, the header module 202 can lack the display screen 206, or the display screen 206 can be detachably connected (removably attachable) to a housing 410 of the header module 202. In such aspects, the header module 202 can be communicably couplable to an external system that is configured to display the information generated by the modules 201 of the modular surgical system 200. For example, in robotic surgical applications, the modular surgical system 200 can be communicably couplable to a robotic cart or robotic control console, which is configured to display the information generated by the modular surgical system 200 to the operator of the robotic surgical system. As another example, the modular surgical system 200 can be communicably couplable to a mobile display that can be carried or secured to a surgical staff member for viewing thereby. In aspects utilizing a user interface that is separate from or otherwise distinct from the modular surgical system 200, the user interface can be wirelessly connectable with the modular surgical system 200 as a whole or one or more modules 201 thereof such that the user interface can display information from the connected modules 200 thereon.

Referring still to FIG. 4A, the energy module 204 can include a port assembly 412 including (providing) a number of different ports configured to deliver different energy modalities to corresponding surgical instruments (e.g., surgical instruments 300, 330, 360 of FIG. 3, for example) that are connectable thereto. In the particular aspect illustrated in FIGS. 4A, the port assembly 412 includes a bipolar port 414, a first monopolar port 416a, a second monopolar port 416b, a neutral electrode port 418 (to which a monopolar return pad is connectable), and a combination energy port 420. However, this particular combination of ports is simply provided for illustrative purposes and alternative combinations of ports and/or energy modalities may be possible for the port assembly 412.

As noted above, the modular surgical system 200 can be assembled into different configurations. Further, the different configurations of the modular surgical system 200 can also be utilizable for different surgical procedure types and/or different tasks. For example, FIGS. 4A and 4B illustrate a first illustrative configuration of the modular surgical system 200 including the header module 202 (including the display screen 206) and the energy module 204 connected together. Such a configuration can be suitable for laparoscopic and open surgical procedures, for example. As shown in FIG. 4B, the modular surgical system 200 can be positioned on a cart 230 enabling the modular surgical system 200 to be easily moved (wheeled) around the operating room, for example.

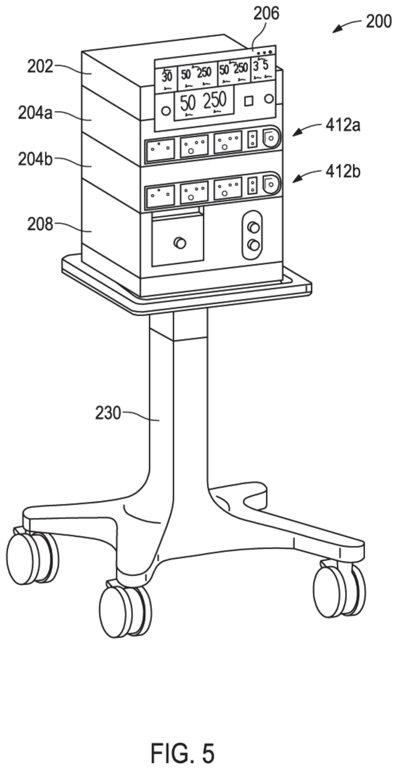

FIG. 5 illustrates a second illustrative configuration of the modular surgical system 200 including the header module 202 (including the display screen 206), a first energy module 204a, a second energy module 204b, and the evacuation module 208 connected together and positioned on the cart 230. In such a configuration, the evacuation module 208 can evacuate smoke, fluid, and/or particulates generated by surgical instruments powered by the energy modules 204a,b.

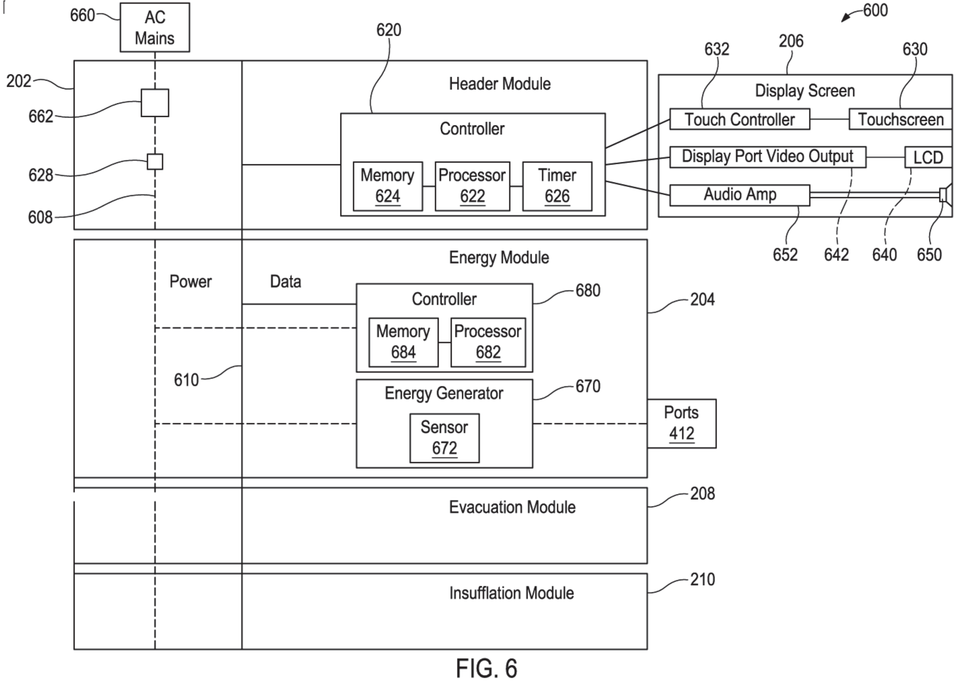

FIG. 6 is a block diagram of an example modular surgical system 600, in accordance with at least one aspect of the present disclosure. As illustrated, the modular surgical system 600 includes the header module 202 (including the display screen 206), the energy module 204 stacked under and coupled to the header module 202, the evacuation module 208 stacked under and coupled to the energy module 204, and the insufflation module 210 stacked under and coupled to the evacuation module 208.

The header module 202 is configured to monitor, control, energize, and provide feedback concerning operation of the modules within the modular surgical system 600, such as the energy module 204, the evacuation module 208, and the insufflation module 210. As illustrated, the header module 202 includes a controller 620 that comprises a processor 622 and a memory 624 storing computer readable instructions executable by the processor 622 to carry out functions and operations of the header module 602. Examples of the memory 624 include, but are not limited to, random access memory (RAM), read-only memory (ROM), computer chips, optical discs (e.g., compact discs (CDs), digital video discs (DVDs), etc.), magnetic disks (e.g., hard disk drives (HDDs), floppy disks, ZIP^{®} disks, etc.), magnetic tape, and solid state storage devices (e.g., memory cards, "flash" media, etc.). As used herein, the term "computer readable medium" refers to any device or system for storing and providing information (e.g., data and instructions) to the processor 622. Examples of computer readable media include, but are not limited to, optical discs, magnetic disks, magnetic tape, solid-state media, and servers for streaming media over networks.

Based on instructions stored in the memory 624, the processor 622 may be configured to control power and data transmissions between the header module 202, the energy module 204, the evacuation module 208, and the insufflation module 210 through a power interface 608 and a data interface 610. For example, the header module 202 can transmit various commands to the energy module 204, the evacuation module 208 (through the energy module 204), and the insufflation module 210 (through the energy module 204 and the evacuation module 208) via the data interface 610. Such commands can be based on user inputs received at the display screen 206 or inputs received by the controller 620 from various sensors communicably coupled to the modular surgical system 600, as discussed elsewhere herein.

As a further example, power may be transmitted to the energy module 204, the evacuation module 208 (through the energy module 204), and the insufflation module 210 (through the energy module 204 and the evacuation module 208) from the header module 202 via the power interface 608. The header module 202 may receive power from an external power source 660 (referred to herein as "AC Mains"), such as a wall outlet, for example. The header module 202 may include an AC/DC converter 662 which receives the AC power from the AC Mains 660 and converts the AC power to DC power. The controller 202 may then distribute the DC power to the energy module 204, the evacuation module 208, and the insufflation module 210 via the power interface 608. The controller 620 may further include a timer 626 for measuring elapsed time. The header module 202 may include a sensor 628, such as a current sensor and/or a power sensor, for example, in operable communication with the controller 620 for measuring current and power along the power interface 608.

As shown in FIG. 6, the energy module 204 may include a controller 680 that comprises a processor 682 and a memory 684 storing computer readable instructions executable by the processor 682 to carry out functions and operations of the energy module 204. The processor 682 and a memory 684 may be similar to processor 622 and memory 624, respectively. The controller 680 may receive power from the AC/DC converter 662 along the power interface 608 and may be in operable communication with controller 620 via the data interface 610.

The energy module 204 may further include an energy generator 670. The energy generator 670 may receive power from the AC/DC converter 662 along the power interface 608 and may be in operable communication with controller 680, such as via a wired or wireless connection. The energy generator 670 may be operable to provide therapeutic energy to one or more surgical instruments, such as the surgical instruments 300, 330, 360, via the port assembly 412, such as via the bipolar port 414 (FIG. 4), the first or second monopolar ports 416a, 416b (FIG. 4), or the combination energy port 420 (FIG. 4), for example. For instance, the energy generator 670 may be energized with DC power provided thereto from the AC/DC converter 662 along the power interface 608. The controller 680 may then receive an input, such as from the controller 620. Based on the input, the controller 680 may control the energy generator 670 to provide therapeutic energy to one or more surgical instruments coupled to the energy module 204 at the port assembly 412. The energy generator 670 may include a sensor 672, such as a current sensor and/or a power sensor, for example, in operable communication with the controller 680 for measuring current and/or power provided by the energy generator 670. The sensor 672 may also comprise an impedance sensor for measuring the impedance of tissue grasped by one of the surgical instruments.

As shown in FIG. 6, the display screen 206 includes a touchscreen 630 coupled to a touch controller 632. The touch controller 632 is coupled to the controller 620 to read inputs, such as user inputs, from the touchscreen 630. The controller 620 drives an LCD display 640 through a display/port video output signal 642. The controller 620 is further coupled to an audio amplifier 652 to drive one or more speakers 650.

### Surgical Insufflation

Minimally invasive surgical procedures often require the creation of a gas-filled cavity to provide surgeons with adequate visibility and space to manipulate instruments, such as the energy delivery devices 300, 330, 360 of FIG. 3. This gas-filled cavity may be generated using an insufflation module, such as insufflation module 210 (FIG. 2).

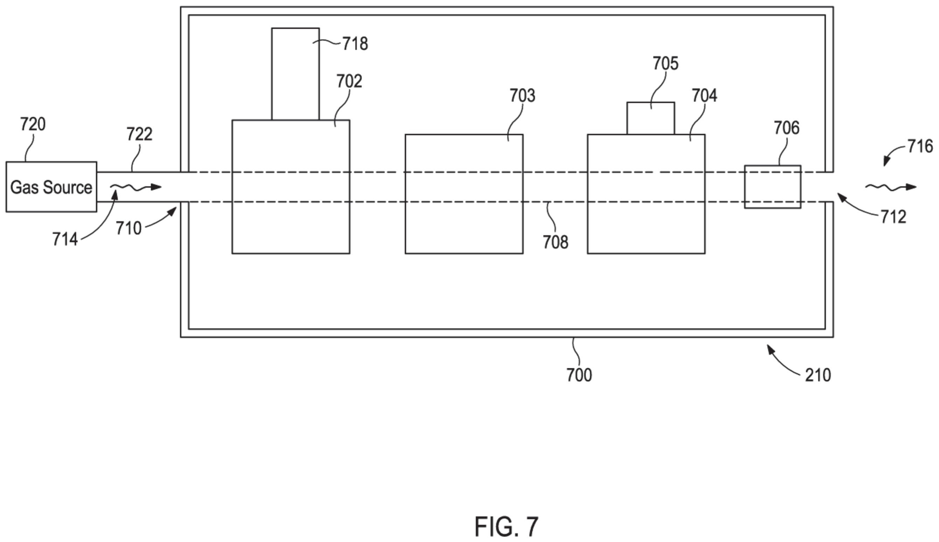

FIG. 7 is a schematic view of the internal components of the insufflation module 210 of FIG. 2, according to at least one aspect of the present disclosure. The insufflation module 210 may include an insufflation housing 700 that contains a fan or pump 702, a humidifier 703, a heat reservoir 704, a heater 705, and an exhaust mechanism 706, all positioned therewithin. In some embodiments, the heater 705 may be operable to generate heat and the heat reservoir 704 is configured to receive and store the heat generated by the heater 705. This stored heat may be used to passively warm gas that moves through the insufflation module 210, as will be described in more detail below. Alternatively, in other embodiments, the heat reservoir 704 may be omitted and the heater 705 may be operable to actively warm gas that moves through the insufflation module 210. The humidifier 703 may be operable to humidify the gas that moves through the insufflation module 210, as will also be described in more detail below. A motor 718 is provided to drive the pump 702. When the insufflation module 210 is stacked with the header module 202, similar to the arrangement shown in FIG. 6, the motor 718, the humidifier 703, and the heater 705 may receive power from the AC/DC converter 662 via the power interface 608.

The insufflation module 210 defines a flow path 708 (shown partially in dashed lines) extending through the insufflation housing 700 and having an inlet port 710 and an outlet port 712. The pump 702, the humidifier 703, the heat reservoir 704, and the exhaust mechanism 706 are sequentially arranged in-line within the flow path 708 through the insufflation housing 700 between the inlet port 710 and the outlet port 712. The outlet port 712 may be fluidically coupled to a trocar, which may be in fluid communication with an internal cavity of a patient (e.g., a patient's peritoneal cavity).

The inlet port 710 may be fluidically coupled to a gas source 720 with a conduit 722 (tubing). The gas source 720 may contain a gas, such as carbon dioxide (CO₂), nitrous oxide (N₂O), helium, oxygen, air, xenon, argon, or nitrogen (N₂), as examples.

The pump 702 is configured to produce a pressure differential in the flow path 708 by a mechanical action. The pressure differential draws gas 714 from the gas source 720 through the conduit 722, into the inlet port 710, and along the flow path 708. After moving through the humidifier 703 and the heat reservoir 704, the gas 714 can be considered "heated/humidified" gas 716 (referred to herein as "insufflation" gas 716), which can continue through the flow path 708 and the exhaust mechanism 706, eventually being expelled (discharged) through the outlet port 712. The exhaust mechanism 706 may control the velocity, the direction, and/or other properties of the insufflation gas 716 exiting the insufflation module 210 at the outlet port 712.

The flow path 708 through the insufflation module 210 can be comprised of a tube or other conduit that substantially contains and/or isolates the fluid moving through the flow path 708 from the fluid (the ambient environment) outside the flow path 708.

### Surgical Smoke Evacuation

As provided herein, energy devices, such as the energy delivery devices 300, 330, 360 of FIG. 3, deliver mechanical (ultrasonic, for example) and/or electrical (RF, for example) energy to target tissue in order to treat the tissue (e.g. to cut the tissue, cauterize blood vessels and/or coagulate the tissue within and/or near the targeted tissue). Cutting, cauterization, and/or coagulation of tissue can result in fluids and/or particulates being released into the air. Such fluids and/or particulates emitted during a surgical procedure can constitute smoke, for example, which can comprise carbon particles and/or other particles suspended in air. In other words, a fluid can comprise smoke and/or other fluidic matter.

Approximately 90% of endoscopic and open surgical procedures generate some level of smoke. The smoke can be unpleasant to the olfactory senses of the clinician(s), the assistant(s), and/or the patient(s), may obstruct the clinician(s)'s view of the surgical site, and may be unhealthy to inhale in certain instances. For example, smoke generated during an electrosurgical procedure can contain toxic chemicals including acrolein, acetonitrile, acrylonitrile, acetylene, alkyl benzenes, benzene, butadiene, butene, carbon monoxide, creosols, ethane, ethylene, formaldehyde, free radicals, hydrogen cyanide, isobutene, methane, phenol, polycyclic aromatic hydrocarbons, propene, propylene, pyridene, pyrrole, styrene, toluene, and xylene, as well as dead and live cellular material (including blood fragments), and viruses. Certain materials identified in surgical smoke have been classified as containing known carcinogens. It is estimated that one gram of tissue cauterized during an electrosurgical procedure can be equivalent to the toxins and carcinogens of six unfiltered cigarettes. Additionally, exposure to the smoke released during an electrosurgical procedure has been reported to cause eye and lung irritation to health care workers.

In addition to the toxicity and odors associated with the material in surgical smoke, the size of particulate in surgical smoke can be harmful to the respiratory system of the clinician(s), the assistant(s), and/or the patient(s). In certain instances, the particulates can be extremely small, and repeated inhalation of extremely small particulate can lead to acute and chronic respiratory conditions in certain instances.

Many electrosurgical systems employ a surgical evacuation system that draws in and captures the resultant smoke from a surgical procedure, and directs the captured smoke through a filter and an exhaust port away from the clinician(s) and/or from the patient(s). For example, an evacuation system, such as the evacuation module 208 (FIG. 2), can be configured to evacuate smoke that is generated during an electrosurgical procedure. Such an evacuation system can be referred to as a "smoke evacuation system," though such evacuation systems can be configured to evacuate more than just smoke from a surgical site.

Throughout the present disclosure, the "smoke" evacuated by an evacuation system is not limited to just smoke. Rather, the smoke evacuation systems disclosed herein can be used to evacuate a variety of fluids, including liquids, gases, vapors, smoke, steam, or combinations thereof. The fluids can be biologic in origin and/or can be introduced to the surgical site from an external source during a procedure. The fluids can include water, saline, lymph, blood, exudate, and/or pyogenic discharge, for example. Moreover, the fluids can include particulates or other matter (e.g. cellular matter or debris) that is evacuated by the evacuation system. For example, such particulates can be suspended in the fluid.

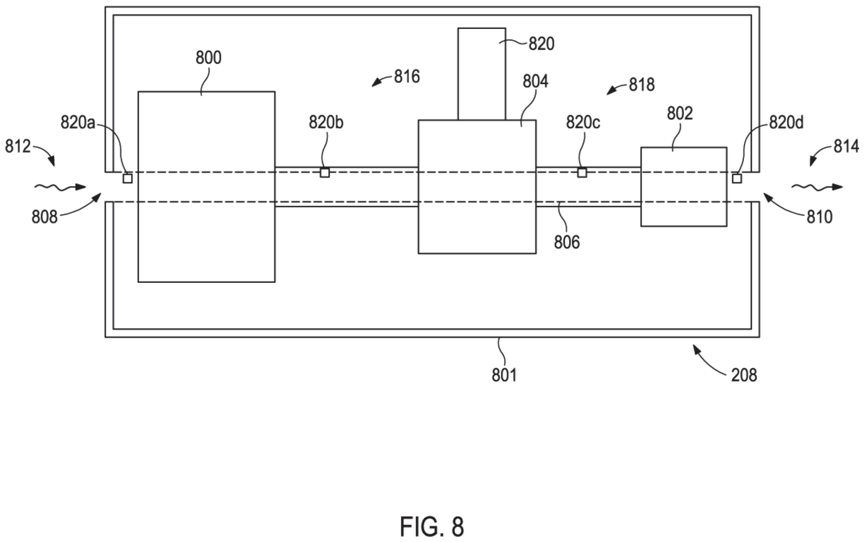

FIG. 8 is a schematic view of the internal components of the evacuation module 208 of FIG. 2, according to at least one aspect of the present disclosure. The evacuation module 208 includes an evacuation housing 801 with a fan or pump 804 and a filter 800 positioned therewithin. Smoke drawn into the evacuation housing 801 travels to the filter 800, and harmful toxins and offensive smells are filtered out of the smoke as it moves through (traverses) the filter 800. Filtered air 814 may then exit the evacuation module 208 as exhaust.

The evacuation module 208 defines a flow path 806 (shown in dashed lines) extending through the evacuation housing 801 and having an inlet port 808 and an outlet port 810. The filter 800, the pump 804, and the exhaust mechanism 802 are sequentially arranged in-line within the flow path 806 through the evacuation housing 801 between the inlet port 808 and the outlet port 810. The inlet port 808 may be fluidically coupled to a suction conduit 702, which can comprise a distal conduit opening positionable at a surgical site.

The pump 804 is configured to produce a pressure differential in the flow path 806 by a mechanical action. The pressure differential is configured to draw smoke 812 from the surgical site into the inlet port 808 and along the flow path 806. After moving through the filter 800, the smoke 812 can be considered "filtered" smoke or air 814 (referred to herein as "filtered air 814"), which can continue through the flow path 806 and is eventually expelled (discharged) through the outlet port 810.

As illustrated, the flow path 806 may include a first zone 816 and a second zone 818. The first zone 816 is located upstream from the pump 804; the second zone 818 is located downstream from the pump 804. The pump 804 is configured to generate the vacuum and otherwise pressurize the fluid in the flow path 806 to drive the fluid from the first zone 816 to the second zone 818 through the pump 804. A motor 820 drives the pump 804. When the evacuation module 208 is stacked with the header module 202, similar to the arrangement shown in FIG. 6, the motor 820 may receive power from the AC/DC converter 662 via the power interface 608. The exhaust mechanism 802 is a mechanism that can control the velocity, the direction, and/or other properties of the filtered air 814 exiting the evacuation module 208 at the outlet port 810.

The flow path 806 through the evacuation module 208 can be comprised of a tube or other conduit that substantially contains and/or isolates the fluid moving through the flow path 806 from the fluid (the ambient environment) outside the flow path 806. For example, the first zone 816 of the flow path 806 can comprise a tube through which the flow path 806 extends between the filter 800 and the pump 804. The second zone 818 of the flow path 806 can also comprise a tube (conduit) through which the flow path 806 extends between the pump 804 and the exhaust mechanism 802. The flow path 806 also extends through the filter 800, the pump 804, and the exhaust mechanism 802 so that the flow path 806 extends continuously from the inlet port 808 to the outlet port 810.

In operation, the smoke 812 can flow into the filter 800 after traversing the inlet port 808 and can be pumped through the flow path 806 by the pump 804 such that the smoke 812 is drawn into the filter 800. The filtered air 814 discharged from the filter 800 can then be pumped through the exhaust mechanism 802 and out the outlet port 810 of the evacuation module 208. The filtered air 814 exiting the evacuation module 208 at the outlet port 810 is the exhaust, and can consist of filtered gases that have passed through the evacuation module 208. Additional information regarding the evacuation module 208 is described in U.S. Patent No. 11,602,393, entitled "SURGICAL EVACUATION SENSING AND GENERATOR CONTROL", issued March 14, 2023, which is hereby incorporated by reference in its entirety herein.

According to embodiments of the present disclosure, the evacuation module 208 may further include a plurality of sensors 820a, 820b, 820c, 820d positioned along the flow path 806 for measuring one or more parameters associated with the smoke 812 and/or the filtered air 814 flowing along the flow path 806. When the evacuation module 208 is in a stacked configuration with a header module 202, such as in the configuration shown in FIG. 6, for example, the sensors 820a-d can be in operable communication with the controller 620, such as along the data interface 610 (FIG. 6), and can receive power from the header module 202 via the power interface 608 (FIG. 6). The controller 620 may receive measurements from the sensors 820a-d and control various operations of the modular surgical system based on these measurements. For example, in some embodiments, the controller 620 can control a speed of the motor 820 based on the received measurements.

One or more of the sensors 820a-d may comprise flow sensors for measuring a flow rate of the smoke 812/filtered air 814 along the flow path 806, such as a flow rate entering the inlet port 808 (sensor 820a), a flow rate through the first zone 816 (sensor 820b), a flow rate through the second zone 818 (sensor 820c), and/or a flow rate exiting the outlet port 810 (sensor 820d), for example. Alternatively, or in addition thereto, one or more of the sensors 820a-d may comprise pressure sensors for measuring a pressure of smoke 812/filtered air 814 along the flow path 806, such as at the inlet port 808 (sensor 820a), the first zone 816 (sensor 820b), the second zone 818 (sensor 820c), and/or at the exit outlet port 810 (sensor 820d), for example. In some embodiments, the sensors 820a-d may be used by the controller 620 to measure a pressure differential along the flow path 806, such as a pressure differential across the filter 800 using the first and second sensors 820a,b. The evacuation module 208 may include a combination of both flow sensors and pressure sensors.

### Look Up Table for Amount of Smoke Produced by different devices

Operating room (OR) staff, such as nurses, typically have to manually adjust multiple settings of insufflators and evacuators, such as run time, pressures, or flow rates, during a surgical procedure as devices are changed and/or different procedural steps are completed. Systems and methods for automatically adjusting settings pre-emptively, or automatically, may provide a more efficient surgical procedure with less technical support needed.

Referring again to FIGS. 6 and 8, the controller 620 may receive an input indicative of a type of a surgical instrument (e.g., one of surgical instruments 300, 330, 360 of FIG. 3) coupled to the energy module 204 and a set power level for the associated surgical instrument. The controller 620 may include, in the memory 624, a look-up table that correlates types of surgical instruments and power levels thereof to motor speeds of the evacuation motor 820. Accordingly, the controller 620 may set motor speeds of the evacuation motor 820 according to a determined type of the surgical instrument and power level thereof.

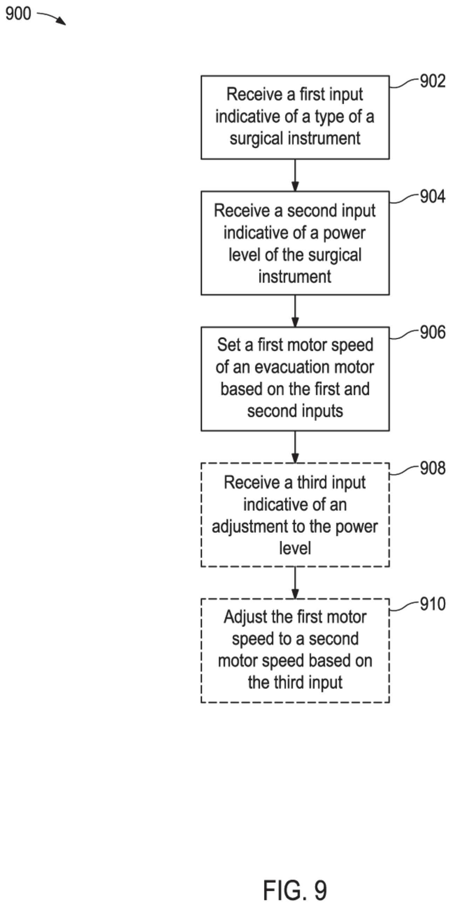

FIG. 9 is a schematic flowchart of an example method 900 of controlling the modular surgical system 600 of FIG. 6, according to at least one aspect of the present disclosure. The method 900 may be embodied as an algorithm, stored in the memory 624 (FIG. 6) of the controller 620 (FIG. 6), and may be executable by the processor 622 (FIG. 6) of the controller 620. Alternatively, the algorithm may be stored in the memory 684 (FIG. 6) of the controller 680 (FIG. 6), and may be executable by the processor 682 (FIG. 6) of the controller 680.

With reference to FIGS. 6 and 9, the method 900 may include receiving a first input indicative of a type of a surgical instrument, as at step 902. For instance, in some embodiments, a user may provide an input to the controller 620 via the touchscreen 630, informing the controller 620 of the type of surgical instrument coupled to, or to be coupled to, the energy module 204. In other embodiments, the user may couple the surgical instrument to the energy module 204 and the controller 620 may receive an input based on the connection. For instance, the controller 620 may identify the instrument as being a bipolar surgical instrument based on the surgical instrument being coupled to the bipolar port 414 (FIG. 4), a monopolar surgical instrument based on the surgical instrument being coupled to one of the first or second monopolar ports 416a,b (FIG. 4), or a combination instrument based on the surgical instrument being coupled to the combination port 420 (FIG. 4).

The method 900 may further include receiving a second input indicative of a power level of the surgical instrument, as at step 904. For instance, based on the controller 620 identifying the type of the surgical instrument, the controller 620 may display, on the display 206, an interactable (interactive) widget that the user may interact with to set and adjust the power level of the surgical instrument. Example widgets are described in U.S. Patent No. 11,666,368, titled "METHOD FOR CONSTRUCTING AND USING A MODULAR SURGICAL ENERGY SYSTEM WITH MULTIPLE DEVICES", which issued June 6, 2023, the contents of which are hereby incorporated by reference in their entirety herein.

The method 900 may further include setting a first motor speed of an evacuation motor based on the first and second inputs, as at step 906. For instance, the memory 624 of the controller 620 may include a look-up table that correlates types of surgical instruments and set power levels thereof with preselected motor speeds of the evacuation motor 820 (FIG. 8). The controller 620 may retrieve from the memory 624 the first motor speed of the evacuation motor 820 based on receiving the first and second inputs. The controller 620 may then set the motor speed of the evacuation motor 820 to the first motor speed. The selected motor speed may be sufficient to evacuate an amount of smoke expected to be generated by the type of the surgical instrument at the set power level. The controller 620 may energize the evacuation motor 820 at the first motor speed via the power interface 608.

The method 900 may optionally further include receiving a third input indicative of an adjustment to the power level, as at step 908, and adjusting the first motor speed to a second motor speed based on the third input, as at step 910. For instance, after the controller 620 has set the first motor speed (step 906), a user may desire to increase or decrease the power level (first power level) of the surgical instrument to a second power level. A user may provide an input to the controller 620 via the touchscreen 630, as described above, informing the controller 620 that a power level adjustment to the surgical instrument is desired. The controller 620 may adjust the first power level of the surgical instrument to a second power level based on the user input, while also retrieving from the memory 624, a second motor speed of the evacuation motor 820 (FIG. 8) for the type of the surgical instrument at the second power level. The selected second motor speed may be sufficient to evacuate an amount of smoke expected to be generated by the type of the surgical instrument at the set second power level. The controller 620 may energize the evacuation motor 820 at the second motor speed via the power interface 608 (FIG. 6).

Accordingly, the modular surgical system 600 may automatically set motor speeds of the evacuation motor 820 (FIG. 8) according to identified types of surgical instruments, and associated power levels thereof, thereby allowing the system to make changes to the system proactively, rather than reactively.

### Interface and software logic for creating an intelligent and interactive system

Referring again to FIG. 6, the controller 620 may utilize patient information, such as a patient's height and weight, to set one or more parameters of surgical modules utilized in the modular surgical system 600, such as the evacuation module 208 or the insufflation module 210. The memory 624 of the controller 620 may include a look-up table that correlates patient information with preselected motor speeds of the evacuation motor 820 (FIG. 8) and/or the insufflation motor 718 (FIG. 7). A user may provide the patient information to the controller 620 via the touchscreen 630 and the controller 620 may retrieve the preselected motor speeds from the memory 624 according to the provided patient information.

A user may desire to adjust the preselected motor speeds retrieved from the look-up table. For instance, the controller 620 may display, on the display 206, the retrieved preselected motor speed as an interactive widget that shows a user the preselected motor speed. The user may interact with the interactive widget to adjust the motor speed, such as increasing or decreasing the motor speed, to a user adjusted motor speed. The controller 620 may include a learning database, such as an artificial intelligence (AI) platform that detects the adjustment of the preselected motor speed to the user adjusted motor speed. Based on the settings being modified by the user, the learning database may self-update the look-up table to provide the desired settings for the specific patient range. The new setting information may be stored in the memory 624 as the new preferred settings, resulting in an ever adjusting/learning data set. Accordingly, the modular surgical system 600 may be referred to as an intelligent and interactive system.

The controller 620 may set the motors 718, 820 (FIGS. 7 and 8, respectively) to the preselected, or user adjusted, motor speeds and may energize the motors 718, 820, via the power interface 608. The controller 620 may selectively energize the motors 718, 820 based on a user input, such as an input to the touchscreen 630, an activation of a surgical instrument, such as one of the surgical instruments 300, 330, 360 (FIG. 3) coupled with the energy module 204, or one or more components of the modular surgical system 600 being powered on, or combinations thereof. For instance, a user may energize an endoscope that is coupled to the visualization module 212 (FIG. 2). The controller 620 may detect the endoscope being powered on, such as via a signal along the data interface 610, and automatically energize one or both of the evacuation module 208 and the insufflation module 210 based on the detection.

### Venturi system for Smoke Evacuation

With continued reference to FIG. 6, it is desirable to optimize power consumption from the AC Mains 660 due to the varying number of components of the modular surgical system 600 which draw power therefrom. The evacuation module 208 and the insufflation module 210 typically require a large amount of power to drive the evacuation pump 804 (FIG. 8) and the insufflation pump 702 (FIG. 7), respectively. With the evacuation module 208, for example, there are different power needs from the AC Mains 660 depending on the operating environment. Laparoscopic surgical procedures typically require a first (lower) amount of smoke evacuation from the evacuation module 208, whereas open surgical procedures typically require a second (larger) amount of smoke evacuation greater than the first amount of smoke evacuation. These insufflation and smoke evacuation needs may be satisfied with a venturi pump, which requires compressed air in lieu of electrical power.

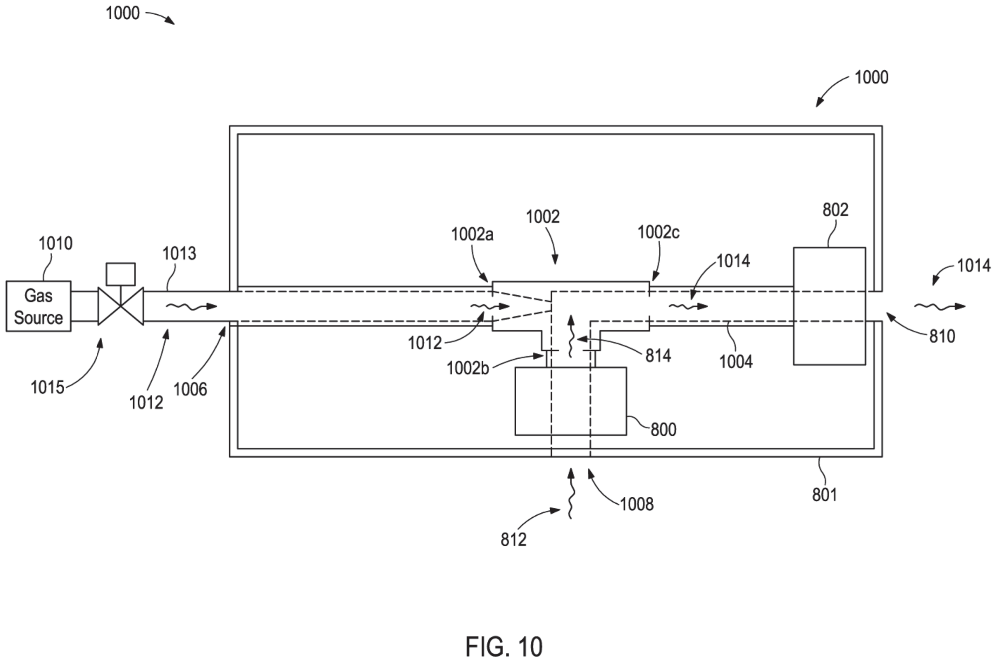

FIG. 10 is a schematic diagram of the internal components of an example evacuation module 1000, according to at least one aspect of the present disclosure. The evacuation module 1000 may be similar in some respects to the evacuation module 208 of FIG. 8, and therefore may be best understood with reference thereto, where like numerals will correspond to like components not described again in detail.

The evacuation module 1000 may include a venturi pump 1002 in lieu of the pump 804 (FIG. 8) and motor 820 (FIG. 8). The evacuation module 1000 may define a "T"-shaped flow path 1004 (shown in dashed lines) extending through the evacuation housing 801 and having a first inlet port 1006, a second inlet port 1008, and an outlet port 810. The first inlet port 1006 may be fluidically coupled to a gas source 1010 which stores therein a gas 1012, such as compressed air or carbon dioxide, as examples. The gas source 1010 may be a gas tank filled with compressed air generated from the insufflation pump 702 (FIG. 7) or a compressed air system of the hospital. The second inlet port 1008 may be fluidically coupled to a suction conduit, which can comprise a distal conduit opening positionable at a surgical site.

In operation, gas 1012 may be provided to the evacuation module 1000 from the gas source 1010 via the first inlet port 1006. For instance, the gas source 1010 may be coupled to the first inlet port 1006 via a conduit 1013 that includes a motor-operated valve 1015 that is configured to control the flow rate of the gas 1012 from the gas source 1010 to the first inlet port 1006. The motor-operated valve 1015 may include any suitable valve (ball, glove, gate, *etc*.) and a motor that is in operable communication with the controller 620 to receive command signals therefrom. The command signals may include transitioning the motor-operated valve 1015 between an open state, where the motor-operated valve 1015 allows gas 1012 to flow to the first inlet port 1006, a closed state, where the motor-operated valve 1015 prevents gas 1012 from flowing to the first inlet port 1006, and one or more intermediate states between the open and closed states, where the motor-operated valve 1015 allows a partial flow of the gas 1012 to the first inlet port 1006. The motor-operated valve 1015 may receive power from the AC Mains 660 or the AC/DC converter 662, for example. In alternative embodiments, a solenoid valve may be utilized in lieu of the motor-operated valve 1015.

The gas 1012 may travel from the first inlet port 1006, through the flow path 1004, and to a first inlet 1002a of the venturi pump 1002. A constriction within the venturi pump 1002 (typically an hourglass shape or a converging conduit) varies the flow characteristics of the gas 1012 traveling through the venturi pump 1002. As the velocity of the gas 1012 into the first inlet 1002a of venturi pump 1002 increases, there is a consequential drop in pressure. This negative pressure drop may draw smoke 812 through second inlet port 1008, along the flow path 1004, through the filter 800 (thereby generating filtered air 814), and to a second inlet 1002b of the venturi pump 1002. The filtered air 814 may be drawn into the flow path 1004 to mix with the gas 1012 within the venturi pump 1002, and the combined filtered air/gas 1014 may be discharged from an outlet 1002c of the venturi pump 1002, along the flow path 1004 to the exhaust mechanism 802, and subsequently discharged from the outlet port 810.

Accordingly, the foregoing evacuation module 1000 may utilize compressed gas in lieu of electric power from the AC Mains 660 to draw smoke 812 from a surgical site, thereby decreasing the power demand from the AC Mains 660. Other embodiments are envisioned in which the venturi pump 1002 is utilized in the insufflation module 210 (FIG. 7) in lieu of the pump 702 (FIG. 7) and motor 718 (FIG. 7) and the insufflation module 210 includes a similar arrangement of elements like the evacuation module 1000 (T-shaped flow path, gas source 1010, motor-operated valve 1015, etc.).

### Smoke Evacuator UV Filter Sterilization

Referring again to FIG. 8, and as described elsewhere herein, the evacuation motor 820 may be energized to drive the pump 804, thereby drawing smoke 812 through the inlet port 808 and to the filter 800. Biological materials from the smoke 812 may be captured by the filter 800. These biological materials may support the growth of bacteria and other harmful organisms that can present a potential health hazard to hospital staff and patients. Accordingly, it may be desirable to kill and/or inhibit the growth of bacteria and other harmful organisms on the filter 800 or other surfaces within the evacuation module 208.

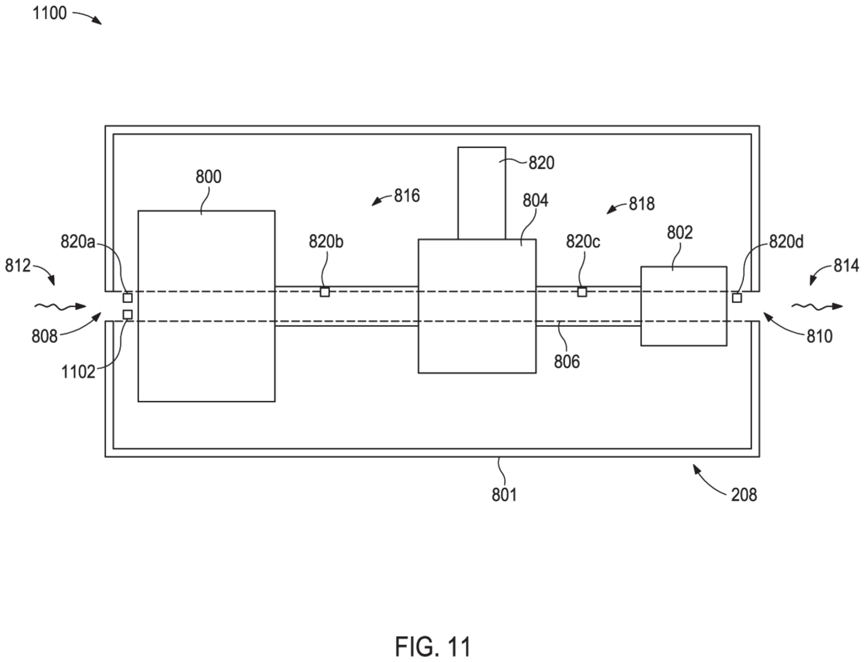

FIG. 11 is a schematic diagram of the internal components of an example evacuation module 1100, according to at least one aspect of the present disclosure. The evacuation module 1100 may be similar in some respects to the evacuation module 208 of FIG. 8, and therefore may be best understood with reference thereto, where like numerals will correspond to like components not described again in detail.

The evacuation module 1100 may include an ultraviolent-C (UV-C) LED 1102 mounted therein, such as to the inlet port 808, to the evacuation housing 801, or at a location along the flow path 806 between the inlet port 808 and the filter 800. The UV-C LED 1102 may be powered (energized) along the power interface 608 when the evacuation module 1100 is in a stacked configuration, similar to the arrangement shown in FIG. 6. The UV-C LED may be energized to kill and/or inhibit the growth of bacteria and other harmful organisms on the filter 800 and/or surfaces within the evacuation module 1100.

The evacuation module 1100 may further include one or more UV transparent windows (not shown) and the UV-C LED 1102 may shine the UV light through the UV transparent windows. The evacuation module 1100 may further includes light-pipes, light fibers, mirrors, or lenses, or combination thereof (not shown) inside the evacuation housing 801 that may be used to distribute the light from the UV-C LED as needed to various surfaces or components inside the evacuation housing 801, thereby killing and/or inhibiting the growth of bacteria and other harmful organisms within the evacuation housing 801.

### Smoke Evacuation utilizing Photelectric Sensor

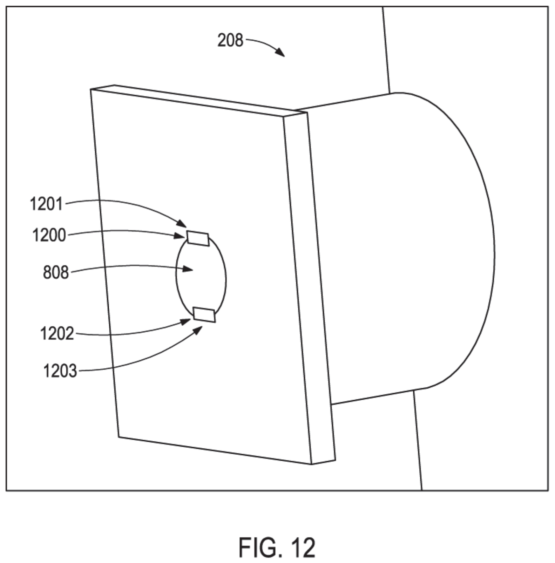

FIG. 12 illustrates a detailed view of the inlet port 808 of the evacuation module 208 of FIG. 8, according to at least one aspect of the present disclosure. The inlet port 808 may include a light transmitter 1200 mounted (coupled) to a first side 1201 of the inlet port 808 and a light receiver 1202 mounted (coupled) to a second side 1203 (i.e., an angularly opposite side) of the inlet port 808 opposite the first side and positioned to confront the light transmitter 1200.

The light transmitter and receiver 1200, 1202 may be powered along the power interface 608 (FIG. 6), and may be in operable communication with the controller 620 (FIG. 6) of the header module (FIG. 6), such as via a wire connection along the data interface 610 (FIG. 6) or wirelessly. The controller 620 may energize the light transmitter 1200, thereby causing light to shine (emit) therefrom toward the light receiver 1202. The controller 620 may determine the quantity of smoke entering the evacuation module 208 based on the amount of light received by the light receiver 1202 from the light transmitter 1200 and control various aspects of the modular surgical system 600 (FIG. 6) based on the determination, as described in more detail below.

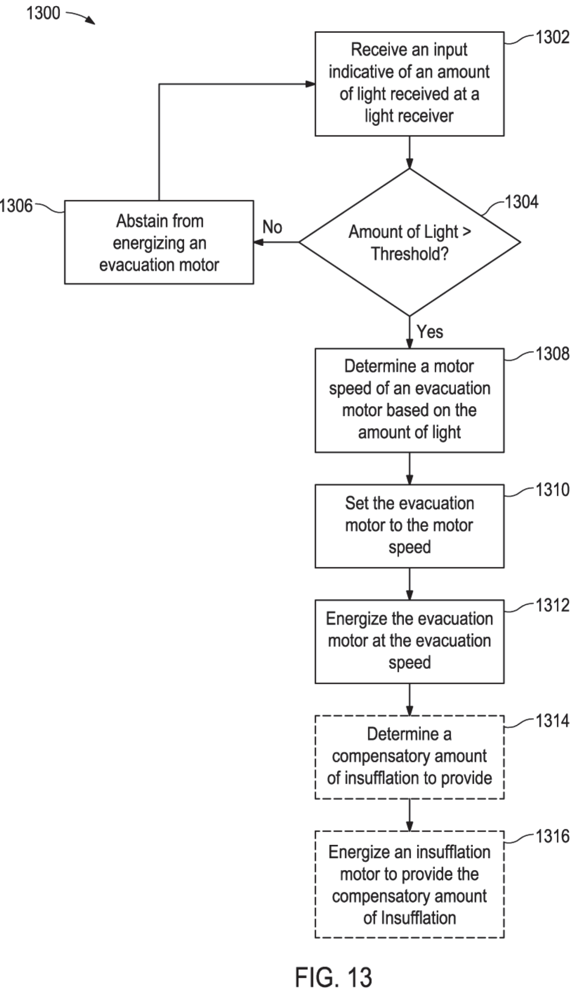

FIG. 13 is a schematic flowchart of an example method 1300 of controlling the modular surgical system 600 of FIG. 6, according to at least one aspect of the present disclosure. The method 1300 may be embodied as an algorithm, stored in the memory 624 (FIG. 6) of the controller 620 (FIG. 6), and may be executable by the processor 622 (FIG. 6) of the controller 620 based on a user providing an input to the controller 620, such as at the touchscreen 630 (FIG. 6).

With reference to FIGS. 6, 8, 12, and 13, the method 1300 may include receiving an input indicative of an amount of light received at a light receiver, as at step 1302. For instance, the controller 620 may energize the light transmitter 1200, thereby causing light to shine (emit) from the light transmitter 1200 toward the light receiver 1202. The light receiver 1202 may receive at least a portion (an amount) of the emitted light from the light transmitter 1200 and the controller 620 may receive an input from the light receiver 1202 indicative of the amount of light received at the light receiver 1202.

The method 1300 may further include comparing the amount of light to a light threshold, as at step 1304. The controller 620 may compare the amount of light to a light threshold, which may be stored in the memory 624 and may be retrievable by the controller 620. In some embodiments, the light transmitter 1200 may be operable to generate a source amount of light, measured in candles, lumens, footcandles, or lux, for example, and the threshold may be a percentage (fraction) of the source amount of light. The light threshold may be 99%, 90%, 80%, 75%, 60%, or 50% of the source amount of light, for example, or any suitable percentage.

The method 1300 may further include abstaining from energizing an evacuation motor, as at step 1306. For instance, based on the amount of light being greater than the light threshold, the controller 620 may abstain from energizing the evacuation motor 820 and loop back to step 1302, as described above.

The method 1300 may further include determining a motor speed of an evacuation motor based on the amount of light, as at step 1308. For instance, based on the amount of light being less than the light threshold, the controller 620 may determine a motor speed of the evacuation motor 820. The memory 624 of the controller 620 may include a look-up table that correlates amounts of light received by the light receiver 1202 to motor speeds of the evacuation motor 820. In the look-up table, it may provide that as the amount of light received at the light receiver 1202 decreases, the motor speed of the evacuation motor 820 should correspondingly increase. For example, a first amount of light may be correlated to a first motor speed and a second amount of light less than the first amount of light may be correlated to a second motor speed greater than the first motor speed. Accordingly, as the amount of light received at the light receiver 1202 diminishes, potentially signifying an increase in smoke between the light transmitter and receiver 1200, 1202, the motor speed of the evacuation motor 820 will correspondingly increase. The controller 620 may retrieve, from the look-up table, the motor speed of the evacuation motor 820 based on the amount of light received by the light receiver 1202.

The method 1300 may further include setting the evacuation motor to the motor speed, as at step 1310, and energizing the evacuation motor at the motor speed, as at step 1312. For instance, the controller 620 may set the evacuation motor 820 to run at the determined motor speed. The controller 620 may then energize the evacuation motor 820 at the determined motor speed via the power interface 608 (FIG. 6).

The method 1300 may optionally further include determining a compensatory amount of insufflation to provide, as at step 1314. The controller 620 may determine a compensatory amount of insufflation to provide to account for the amount of pressure lost due to smoke evacuation, as described above. The controller 620 may determine the compensatory amount of insufflation to provide based on a flow rate of smoke 812 entering the inlet port 808, which may be measured by the flow sensor 820a in cm³/sec, for example, and an elapsed amount of time the evacuation motor 820 is energized, which may be measured by the timer 626 in seconds, for example. The compensatory amount of insufflation to provide may be a volume of insufflation gas 716 (FIG. 7), which may be measured in cm³, for example.

The method 1300 may optionally further include energizing an insufflation motor to provide the compensatory amount of insufflation, as at step 1314. For instance, the controller 620 may energize the insufflation motor 702 (FIG. 7) to drive insufflation gas 716 (FIG. 7) from the insufflation module 210, such as to the patient from which the smoke 812 was drawn from, in order to regulate the pressure within the patient. The controller 620 may maintain the insufflation motor 702 energized until the compensatory amount of insufflation is provided. Based on the compensatory amount of insufflation being provided, the controller 620 may deenergize the insufflation motor 702. In some embodiments, the controller 620 may provide the compensatory amount of insufflation after the evacuation motor 820 has been de-energized and stopped evacuating smoke 812 from the patient. In other embodiments, the controller 620 may provide the compensatory amount of insufflation as the evacuation motor 820 is evacuating smoke 812 to maintain the pressure balance within the patient.

### Automatic Smoke Evacuation - Vision contrast

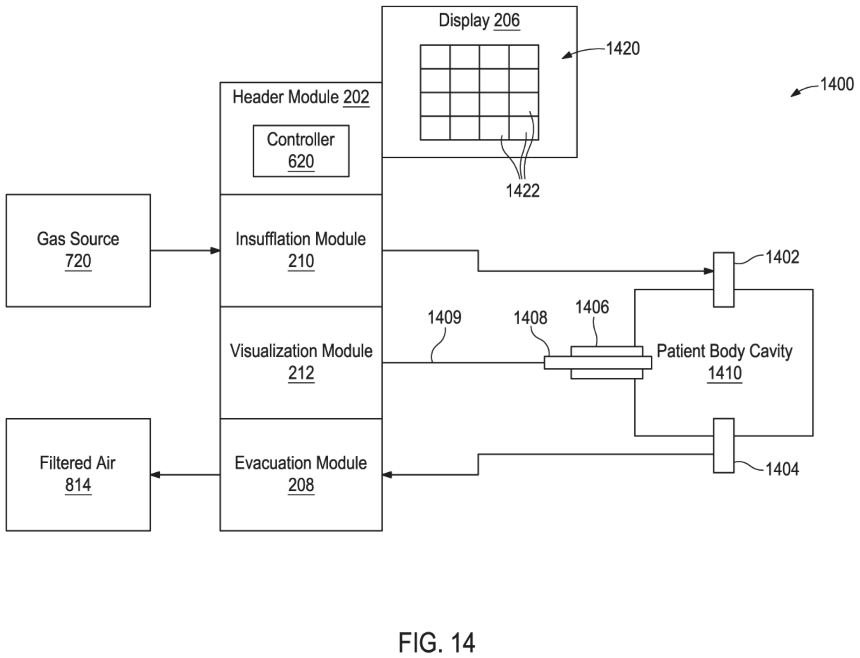

FIG. 14 is a block diagram of another example modular surgical system 1400, in accordance with at least one aspect of the present disclosure. The modular surgical system 1400 may be similar in some respects to the modular surgical system 600 of FIG. 6, and therefore may be best understood with reference thereto. As illustrated, for example, the modular surgical system 1400 may include the header module 202 that includes the controller 620, the display screen 206 coupled to the header module 202, the insufflation module 210 stacked under and coupled to the header module 202, the visualization module 212 stacked under and coupled to the insufflation module 210, and the evacuation module 208 stacked under and coupled to the visualization module 212. The modular surgical system 1400 may include additional modules, described elsewhere herein, such as the energy module 204, for example.

The modular surgical system 1400 may further include an insufflation trocar 1402 inserted into a patient's body cavity 1410 that may be in fluid communication with the insufflation module 210 to receive insufflation gas 716 (FIG. 7) therefrom. The modular surgical system 1400 may further include an evacuation trocar 1404 inserted into the patient's body cavity 1410 that may be in fluid communication with the evacuation module 208 to draw (evacuate) smoke 812 (FIG. 8) from the patient's body cavity 1410. The modular surgical system 1400 may further include a visualization trocar 1406 inserted into a patient's body cavity 1410 that may be sized to receive an endoscope (camera) 1408 therethrough for visualizing a surgical site within the patient body cavity 1410.

While the modular surgical system 1400 is depicted as have three separate trocars 1402, 1404, 1406, other embodiments are envisioned in which the visualization trocar 1406 is omitted and the endoscope 1408 is introduced into the patient body cavity 1410 via the insufflation trocar 1402, for example, and the insufflation gas 716 is provided to the patient body cavity 1410 via an auxiliary connection in the insufflation trocar 1402, such as a luer lock connection, for example.

In operation, the endoscope 1408 may be operable to capture a surgical image, such as a live feed, of a surgical site within the patient body cavity 1410. The imaging data may be communicated from the endoscope 1408 to the visualization module 212, such as via a fiber optic cable 1409 or wirelessly. The imaging data may then be communicated to the controller 620 of the header module 202, such as via the data interface 610 (FIG. 6). Based on receiving the imaging data, the controller 620 may display the surgical image 1420 on the LCD 640 (FIG. 6) of the display 206, thereby allowing operating room (OR) staff to view the surgical site in the patient body cavity 1410 in real time.

During a surgical procedure, a surgical instrument, such as one of the surgical instruments 300, 330, 360 of FIG. 3, may be inserted into the patient body cavity 1410 via a trocar, such as one of trocars 1402, 1404, 1406, to operate on tissue, such as at the surgical site being visualized by the endoscope 1408. Operating on the tissue may cause smoke 812 (FIG. 8) to be generated, thereby occluding the field of view (FOV) of the endoscope 1408. Systems and methods for automatically controlling the evacuation module 208 to draw the smoke from the FOV of the endoscope 1408 are desired.

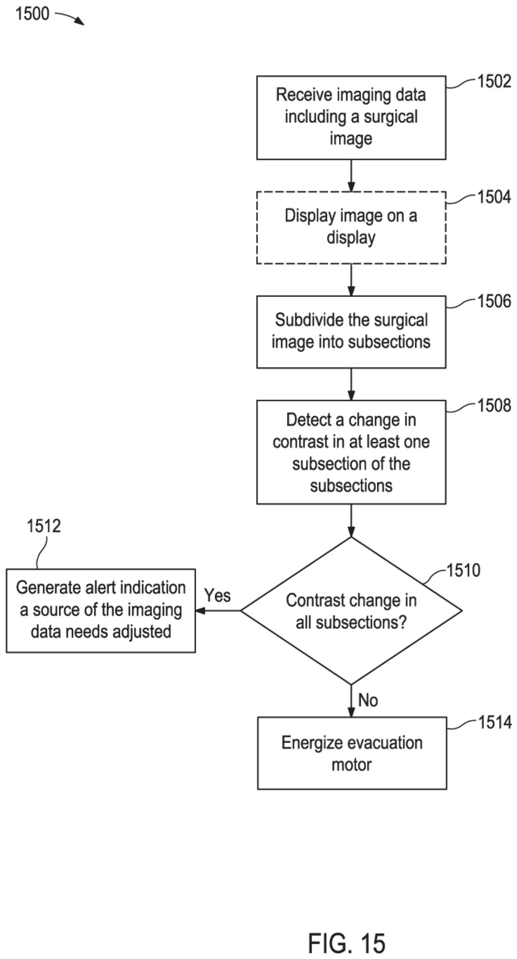

FIG. 15 is a schematic flowchart of an example method 1500 of controlling the modular surgical system 1400 of FIG. 14, according to at least one aspect of the present disclosure. The method 1500 may be embodied as an algorithm, stored in the memory 624 (FIG. 6) of the controller 620 (FIG. 14), and may be executable by the processor 622 (FIG. 6) of the controller 620 based on a user providing an input to the controller 620, such as at the touchscreen 630 (FIG. 6) of the display 206 (FIG. 14).

With reference to FIGS. 6, 14, and 15, the method 1500 may include receiving imaging data including a surgical image, as at step 1502. For instance, the controller 620 may receive imaging data that includes a surgical image from the endoscope 1408, as described herein above. The surgical image may be an image of a surgical site within the patient body cavity 1410, for example.

The method 1500 may optionally further include displaying the surgical image on a display, as at step 1504. For instance, as described herein above, controller 620 may display the surgical image 1420 on the LCD 640 (FIG. 6) of the display 206, thereby allowing operating room (OR) staff to view the surgical site in the patient body cavity 1410 in real time.

The method 1500 may further include subdividing the surgical image into subsections, as at step 1506. For instance, the controller 620 may divide the surgical image into a plurality of subsections 1422, such as a 4x4 grid as shown in FIG. 14, for example. The number of subsections to divide the surgical image 1420 into may be stored in the memory 624. While a 4x4 grid is shown and described, any suitable number and sizes of subsections may be used, such as a 6x6 grid, a 10x10 grip, or a 25x25 grid, as examples.

The method 1500 may further include detecting a change in contrast in at least one subsection of the subsections, as at step 1508. For instance, the controller 620 may utilize Fast Fourier Transform (FFT) to evaluate the contrast of each subsection 1422 and detect a change in the contrast of the subsections 1422 using the same.

The method 1500 may further include determining if there has been a change in contrast in all the subsections, as at step 1510. For instance, based on detecting a change in at least one subsection 1422 of the surgical image 1420, the controller 620 may proceed to evaluate each of the subsections 1422 to determine if each of the subsections 1422 has had a change in contrast, such as via FFT.

The method 1500 may further include generating alert indicating that a source of the imaging data needs adjusted, as at step 1512. For instance, based on the controller 620 determining that the contrast of all the subsections 1422 have changed, the controller 620 may conclude that the image 1420 is out of focus, which may have caused the change in contrast of each of the subsections 1422. Accordingly, the controller 620 may generate an alert, such as an audible alert via the speakers 650 (FIG. 6) or a visual alert via the LCD (FIG. 6), informing the OR staff that the endoscope 1408 should be adjusted, such as refocused.

The method 1500 may further include energizing an evacuation motor, as at step 1514. For instance, based on the controller 620 determining that the contrast of only a portion (less than all) of the subsections 1422 has changed, the controller 620 may conclude that smoke 812 (FIG. 8) is present within the patient body cavity 1410, thereby occluding the FOV. Accordingly, the controller 620 may energize the evacuation motor 820 (FIG. 8) to draw (evacuate) smoke from the patient body cavity 1410, such as via the evacuation trocar 1404. The controller 620 may set the speed of the evacuation motor 820 according to techniques described elsewhere herein.

Accordingly, the controller 620 may monitor a surgical image captured by a camera (e.g., the endoscope 1408) and determine whether changes in the image are due to the camera being out of focus or due to the presence of smoke. Based on the determination, the controller 620 may select an appropriate course of action, such as indicating to OR staff that the camera is out of focus or energizing an evacuation motor.

Embodiments disclosed herein include:
A. A surgical system comprising an evacuation module that includes a motor and a pump drivable by the motor to draw smoke from a patient and a controller operable to receive a first input indicative of a type of a surgical instrument, receive a second input indicative of a power level of the surgical instrument, and set a motor speed of the motor based on the first and second inputs.
B. A surgical system comprising an energy module operably couplable with a surgical instrument, an evacuation module positionable in a stacked configuration with the energy module, the evacuation module including a motor, a pump drivable by the motor to draw smoke from a patient, and a controller, operable to detect the surgical instrument being coupled with the energy module, determine a type of the surgical instrument based on the detection, receive an input indicative of a power level of the surgical instrument, and set a motor speed of the motor based on the type of the surgical instrument and the power level.
C. A method comprising receiving a first input indicative of a type of a surgical instrument, receiving a second input indicative of a power level of the surgical instrument, and setting a motor speed of an evacuation motor based on the type of the surgical instrument and the power level of the surgical instrument.

Each of embodiments A-C may have one or more of the following additional elements in any combination: Element 1: wherein the motor speed is a first motor speed and the controller is further operable to receive a third input indicative of an adjustment to the power level of the surgical instrument and adjust the first motor speed to a second motor speed different than the first motor speed based on the third input. Element 2: wherein the controller includes a memory that stores a look-up table, and wherein the controller is further operable to retrieve, from the look-up table, the motor speed based on the first and second inputs. Element 3: further comprising a display, wherein the controller is operable to receive the first and second inputs via the display. Element 4: further comprising an energy module operable to provide power to the surgical instrument, the controller being operable to receive the first input based on the surgical instrument being coupled to the energy module, and a display, wherein the controller is operable to receive the second input via the display. Element 5: wherein the surgical instrument comprises an ultrasonic surgical instrument. Element 6: wherein the surgical instrument comprises a radio frequency (RF) surgical instrument. Element 7: wherein the motor speed is a first motor speed and the controller is further operable to receive a third input indicative of an adjustment to the power level of the surgical instrument and adjust the first motor speed to a second motor speed different than the first motor speed based on the third input. Element 8: wherein the controller includes a memory that stores a look-up table, the controller being further operable to retrieve, from the look-up table, the motor speed based on the type of the surgical instrument and the power level. Element 9: further comprising a display, wherein the controller is operable to receive the input via the display. Element 10: wherein the energy module comprises a plurality of ports and detecting the surgical instrument being coupled with the energy module comprises detecting the surgical instrument being coupled with a port of the plurality of ports and determining the type of the surgical instrument comprises determining a type of the port to which the surgical instrument is coupled to. Element 11: wherein the surgical instrument comprises an ultrasonic surgical instrument. Element 12: wherein the surgical instrument comprises a radio frequency (RF) surgical instrument. Element 13: wherein the motor speed is a first motor speed, and wherein the method further comprises receiving a third input indicative of an adjustment to the power level of the surgical instrument and adjusting the first motor speed to a second motor speed different than the first motor speed based on the third input. Element 14: wherein receiving the first input indicative of the type of a surgical instrument comprises detecting the surgical instrument being coupled with an energy module and determining the type of the surgical instrument based on the detection. Element 15: wherein detecting the surgical instrument being coupled with the energy module comprises detecting the surgical instrument being coupled with a port of a plurality of ports and determining the type of the surgical instrument comprises determining a type of the port to which the surgical instrument is coupled to. Element 16: wherein receiving the second input indicative of the power level of the surgical instrument comprises receiving the second input indicative of the power level of the surgical instrument via a display.

By way of non-limiting example, exemplary combinations applicable to A, B, and C include: Element 1 with Element 2: Element 1 with Element 3; Element 1 with Element 4; Element 1 with Element 5; Element 1 with Element 6; Element 1 with two or more of Elements 1-6; Element 2 with Element 3; Element 2 with Element 4; Element 2 with Element 5; Element 2 with Element 6; Element 2 with two or more of Elements 2-6; Element 3 with Element 4; Element 3 with Element 5; Element 3 with Element 6; Element 3 with two or more of Elements 1, 2, and 4-6; Element 4 with Element 5; Element 4 with Element 6; Element 4 with two or more of Elements 1-3, 5, and 6; Element 5 with Element 6; Element 5 with two or more of Elements 1-4 and 6; Element 7 with Element 8: Element 7 with Element 9; Element 7 with Element 10; Element 7 with Element 11; Element 7 with Element 12; Element 7 with two or more of Elements 7-12; Element 8 with Element 9; Element 8 with Element 10; Element 8 with Element 11; Element 8 with Element 12; Element 8 with two or more of Elements 8-12; Element 9 with Element 10; Element 9 with Element 11; Element 9 with Element 12; Element 9 with two or more of Elements 7, 8, and 10-12; Element 10 with Element 11; Element 10 with Element 12; Element 10 with two or more of Elements 7-9, 11, and 12; Element 11 with Element 12; Element 11 with two or more of Elements 7-10 and 12; Element 13 with Element 14; Element 13 with Elements 14 and 15; Element 13 with Element 16; Element 13 with two or more of Elements 14-16; Element 14 with Element 15; Element 14 with Element 16; Element 14 with Elements 14 and 16; Element 14 with two or more of Elements 13, 15, and 16; Element 16 with two or more of Elements 13-15.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces. If there is any conflict in the usages of a word or term in this specification and one or more patent or other documents that may be incorporated herein by reference, the definitions that are consistent with this specification should be adopted.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

The use of directional terms such as above, below, upper, lower, upward, downward, left, right, and the like are used in relation to the illustrative embodiments as they are depicted in the figures, the upward direction being toward the top of the corresponding figure and the downward direction being toward the bottom of the corresponding figure.

## Claims

1. A surgical system, comprising:
an evacuation module that includes a motor and a pump drivable by the motor to draw smoke from a patient; and
a controller operable to:
receive a first input indicative of a type of a surgical instrument;
receive a second input indicative of a power level of the surgical instrument; and
set a motor speed of the motor based on the first and second inputs.

2. The surgical system of Claim 1, wherein the motor speed is a first motor speed and the controller is further operable to:
receive a third input indicative of an adjustment to the power level of the surgical instrument; and
adjust the first motor speed to a second motor speed different than the first motor speed based on the third input.

3. The surgical system of Claim 1 or Claim 2, wherein the controller includes a memory that stores a look-up table, and wherein the controller is further operable to retrieve, from the look-up table, the motor speed based on the first and second inputs.

4. The surgical system of any one of Claims 1 to 3, wherein the surgical instrument comprises an ultrasonic surgical instrument.

5. The surgical system of any one of Claims 1 to 3, wherein the surgical instrument comprises a radio frequency (RF) surgical instrument.

6. The surgical system of any one of Claims 1 to 5, further comprising a display, wherein the controller is operable to receive at least the second input via the display.

7. The surgical instrument of Claim 6, wherein the controller is further operable to receive the first input via the display.

8. The surgical system of any one of Claims 1 to 6, further comprising an energy module couplable to the surgical instrument, the controller being operable to receive the first input based on detecting the surgical instrument being coupled to the energy module.

9. The surgical instrument of Claim 8, wherein the energy module is operable to provide power to the surgical instrument.

10. The surgical instrument of Claim 8 or Claim 9, wherein the evacuation module is positionable in a stacked configuration with the energy module.

11. The surgical system of any one of Claims 8 to 10, wherein the energy module comprises a plurality of ports and:
detecting the surgical instrument being coupled with the energy module comprises detecting the surgical instrument being coupled with a port of the plurality of ports; and
determining the type of the surgical instrument comprises determining a type of the port to which the surgical instrument is coupled to.

12. A computer-readable medium comprising instructions which, when executed by a computer, cause the computer to:
receive a first input indicative of a type of a surgical instrument;
receive a second input indicative of a power level of the surgical instrument;
and
set a motor speed of an evacuation motor based on the type of the surgical instrument and the power level of the surgical instrument.

13. The computer-readable medium of Claim 12, wherein the motor speed is a first motor speed, and wherein the method further comprises:
receiving a third input indicative of an adjustment to the power level of the surgical instrument; and
adjusting the first motor speed to a second motor speed different than the first motor speed based on the third input.

14. The computer-readable medium of Claim 12 or Claim 13, wherein receiving the first input indicative of the type of a surgical instrument comprises:
detecting the surgical instrument being coupled with an energy module; and
determining the type of the surgical instrument based on the detection;
optionally wherein detecting the surgical instrument being coupled with the energy module comprises:
detecting the surgical instrument being coupled with a port of a plurality of ports; and
determining the type of the surgical instrument comprises determining a type of the port to which the surgical instrument is coupled to.

15. The computer-readable medium of any one of Claims 12 to 14, wherein receiving the second input indicative of the power level of the surgical instrument comprises receiving the second input indicative of the power level of the surgical instrument via a display.
